# EUROPEAN PATENT SPECIFICATION

(11) **EP 4 245 324 B1**
(45) Date of publication and mention of the grant of the patent: **06.05.2026**
(21) Application number: 23161102.1
(22) Date of filing: 10.03.2023
(51) Int. Cl.: A61M 39/18, A61L 2/10, A61L 2/24, A61M 25/00

(54) **HAND-HELD STERILE CONNECTION DEVICE, SYSTEM, AND METHOD OF USE**
STERILE HANDVERBINDUNGSVORRICHTUNG, SYSTEM UND VERFAHREN ZUR VERWENDUNG
DISPOSITIF DE CONNEXION STÉRILE PORTATIF, SYSTÈME ET PROCÉDÉ D'UTILISATION

(30) Priority: 14.03.2022 US 202263319635 P
(43) Date of publication of application: 20.09.2023
(73) Proprietor: Fenwal, Inc., Lake Zurich, IL 60047 (US)
(72) Inventor: MADSEN, James, Lake Zurich, 60047 (US); WEGENER, Christopher J., Lake Zurich, 60047 (US)
(74) Representative: Maikowski & Ninnemann Patentanwälte Partnerschaft mbB

(56) References cited:
- EP-A1- 0 459 599
- EP-B1- 2 854 873
- WO-A1-2010/036617
- WO-A1-2018/013581
- US-A1- 2006 180 526
- US-A1- 2015 258 230

## Description

### Field of the Disclosure

The present disclosure is directed to a hand-held sterilization device, system, and a method for transferring fluid in a sterile manner. More particularly, the present disclosure is directed to a hand-held sterilization device and system, and a method for creating temporary sterile fluid flow path connections.

### Background

Fluid flow processing systems or assemblies for transferring or dispensing a fluid (liquid) from a source container to one or more receiving containers or receptacles are used in a variety of medical and non-medical applications. In the medical field, in particular, the transfer of fluid must be carried out in a sterile manner, i.e., without exposing the fluids or the open ends of the tubing that carries the fluid to the outside environment. One example of this is the collection of multiple samples from a large volume of a collected biological fluid.

Currently, sterile connections are typically made by joining the ends of connecting tubing associated with a respective part of a processing system, for instance, by cutting, then melting the tubes together creating a fluid pathway, and then sterilizing the pathway.

One example of a device that creates a sterile connection is the TSCD-II sterile tubing welder, available from Terumo Medical Corporation. This device uses a heated cutting element to sever and melt the ends of tubing using a heated wafer, which are joined together after the cutting element is removed. The heated wafer creates an aseptic connection. Aspects of this device are disclosed in U.S. Patent Application Publication No. 2020/0047423.

One disadvantage of welding devices and the traditional methods of making a sterile connection is that such a connection cannot be easily undone as the tubes are physically and permanently bonded. For example, where a fluid from a single source container must be dispensed to multiple (smaller volume) containers or receptacles, each transfer from the source container would require severing the first connection between the source container to the receiving containers, followed by the (re)establishing a new sterile connection between the source and the next receiving container. This sequence of creating a sterile connection, transferring fluid, severing the connection, and re-establishing a sterile connection with the next receiving container is time-consuming, inefficient, and wasteful.

Furthermore, currently available tubing welding devices are benchtop devices that require connections to be made wherever the device is located and not necessarily where the transfer of fluid is to occur. Large, benchtop models are not well suited to be moved from place to place. While there are handheld sterilization devices are known, they mostly are only applicable for sterilizing a single end of a tube or catheter. WO 2018/013581 A1 refers to a handheld and portable disinfection device for use with patients having indwelling catheters hospitalized or in an intensive care unit. WO 2010/036617 A1 refers to a portable photodynamic disinfection light deliver device for catheter disinfection US 2015/258230 A1 refers to a UV-C catheter hub sterilization and data acquisition system. EP 2854873 B1 refers to a UV disinfection system.

Thus, it would be desirable to provide a device which can easily and repeatedly establish a sterile fluid pathway without the successive cutting, heating and physical connecting of plastic tubes. It would also be desirable to provide a portable device that can establish the sterile connection where the fluid transfer is to occur (rather than transport a fluid processing set to a stationary sterile connection device). The device disclosed herein addresses these and other needs.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a hand-held sterilization device is disclosed. The hand-held sterilization device includes a sterilization chamber defined by a base and a lid and at least one holder within the sterilization chamber. The device includes a handle supporting the sterilization chamber, a light source associated with the sterilization chamber, and an actuator configured to move the at least one holder.

In another aspect, a system for providing a sterile fluid pathway connection is disclosed. The system includes a first reusable tube connector configured to receive a first tube and a second reusable tube connector configured to receive a second tube and a hand-held sterilization device. The sterilization device of the system includes a sterilization chamber defined by a base and a lid. The chamber includes at least one holder, configured to receive one of the first or second reusable connectors. The hand-held sterilization device includes a handle supporting the sterilization chamber, a light source associated with the sterilization chamber, and an actuator configured to move the at least one holder.

In yet another aspect, a method for transferring fluid in a sterile manner is disclosed. The method includes attaching an open first end of a tube to a first tube connector wherein the tube includes a second end in flow communication with a source of fluid, and attaching an open first end of a second tube to a second tube connector. The first and second connectors are located within a sterilization chamber carried by a handle of a hand-held device, wherein the chamber receives light from a light source associated with the device. The method further includes temporarily joining the first and second connectors to establish a flow path between the first and second tubes, exposing at least the flow path to a sterilizing light from said light source for a selected period of time, opening the flow path and flowing fluid from said source of fluid through the flow path, and separating the first and second connectors.

### Brief Description of the Drawings

Figure 1 is a perspective view of a sterilization device with a lid in a closed configuration in accordance with the present disclosure;
Figure 2 is a plan view of a first and second connector;
Figure 3 is a perspective view of a sterilization device with a lid in an open configuration in accordance with the present disclosure;
Figure 4 is a flowchart setting forth the steps in the method of creating a sterile connection according to an aspect of the present disclosure;
Figure 5 is a perspective view of a sterilization device with mounted connectors in accordance with the present disclosure;
Figure 6A is a perspective view of the sterilization device with the actuator in a first position, in accordance with the present disclosure;
Figure 6B is a perspective view of the sterilization device with the actuator in a second position, in accordance with the present disclosure;
Figure 7A is a top perspective view of the sterilization chamber of the sterilization device with the connectors mounted in the holders when the actuator is in a first position of Fig. 6A; and
Figure 7B is a top perspective view of the sterilization chamber of the sterilization device with the connectors mounted in the holders when the actuator is in the second position of Fig. 6B.

### Detailed Description of the Embodiments

Insofar as the term embodiment or aspect or alternative is used in the following, or features are presented as being optional, this should be interpreted in such a way that the only protection sought is that of the invention claimed and defined in the appended claims.

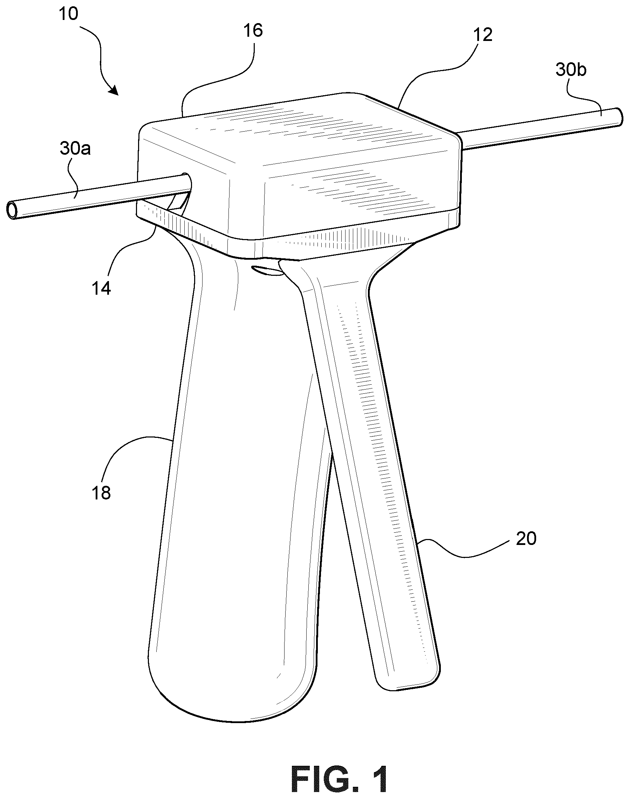

Fig. 1 shows a hand-held sterilization device 10 in accordance with the present disclosure. The sterilization device 10 includes a sterilization chamber 12. The sterilization chamber 12 is defined by a base 14 and a lid 16. The sterilization device 10 further includes an elongated handle 18 and an actuator 20 configured for one-handed operation by user. The hand-held nature of the sterilization device 10 allows a user to transport the device 10 to wherever a sterile connection needs to be made. As opposed to large table-top connection devices, a portable device as disclosed herein provides greater flexibility in choosing a location within a hospital, blood center, or laboratory, for performing the sterile transfer of fluids.

Sterilization device 10 may be used to sterilize reusable connectors shown and described below. In general, the reusable connectors may be attached to tubing associated with two different parts of a fluid kit, circuit, or system. Using reusable connectors allows a user to make a temporary fluid connection between two tubes without physically welding the tubes together or otherwise making an irreversible permanent connection. Generally, the tubing and fluid kit associated with the respective tubing, including the pre-attached reusable connector, will be sterilized prior to making a connection.

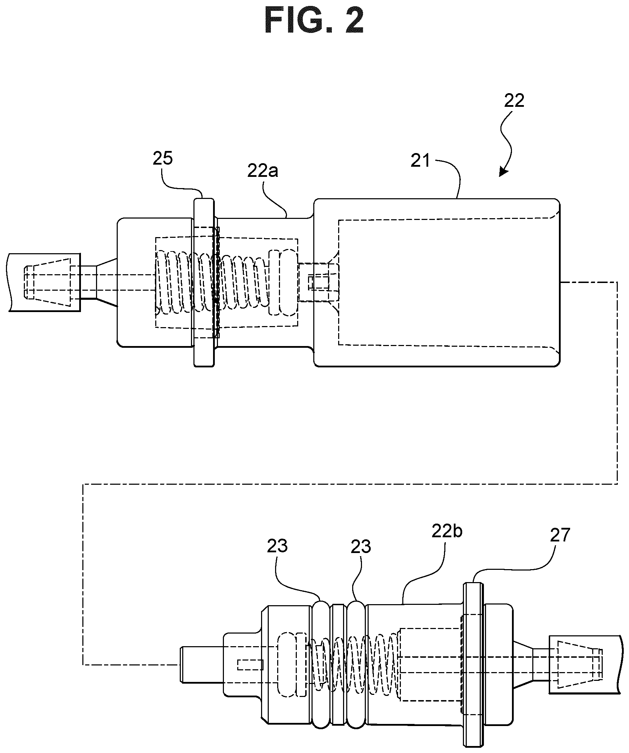

Fig. 2 shows a first and second reusable connector, 22a and 22b, respectively. The connectors are configured to allow for attachment of open tubing ends to the connectors to create a fluid pathway between the tubing when the connectors are temporarily secured to one another. In one embodiment, the reusable connectors 22a and 22b may be attached to a tube using a barb or other interference-type fitting. Other methods known in the art may be used to connect the reusable connectors to tubing without departing from the scope of the disclosure. For instance, the reusable connectors 22a and 22b may be connected by, for example, but not limited to, using a luer fitting or gluing the tube to the end of the reusable connector.

The outer surfaces of reusable connectors may be, but are not limited to being, made of a transparent material. In one embodiment, the reusable connectors are made of a transparent material that can be penetrated by light from the sterilizing light source. In a more particular embodiment, the material may be transparent to at least UV-C light. In another embodiment, the reusable connectors may be made of a cyclic olefin copolymer (COC). Other transparent materials known in the art may be used without departing from the scope of the disclosure.

As further seen in Fig. 2, the first and second reusable connectors 22a and 22b are a female and male connectors, respectively. Reusable connectors 22a, 22b include spring activated plungers movably disposed within each connector, respectively. In an uncompressed state, the spring plunger presses a sealing member such as an O-ring, acting as a face seal, against the respective opening of each connector 22a and 22b to close the fluid path within each connector 22a, 22b. Upon joining the first and second connectors 22a and 22b, O-rings 23 associated with the outside of the second reusable connector 22b create an air-tight seal within a receiving chamber 21 of the first reusable connector 22a configured to receive the second reusable connector 22b. Upon pressing the connectors closer together, spring-activated plungers of both facing connectors 22a and 22b align and push against each other compressing and thereby opening the O-ring face seals of each connector 22a, 22b to open the fluid path. A more detailed description of connectors and their operation is set forth in U.S. Patent Publ. 2023/0285734 A1 "Reversible Sterile Connection System" filed simultaneously herewith.

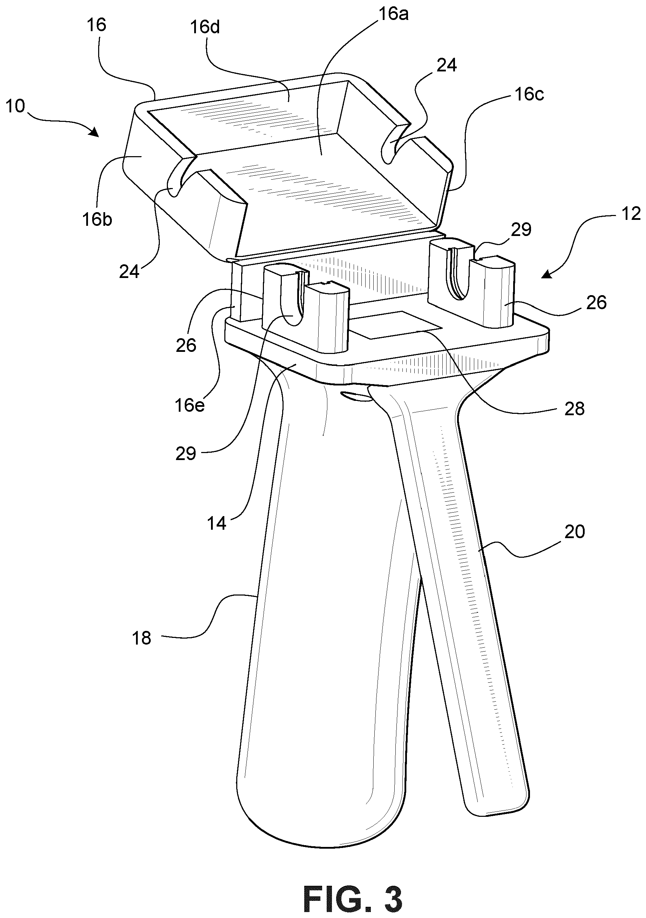

Fig. 3 shows a hand-held sterile connection device 10 with lid 16 in an open configuration. In one embodiment, lid 16 may include a top wall or roof 16a and sidewalls 16b, 16c, 16d and 16e. As shown in Fig. 3, at least side walls 16b, 16c and 16d depend downwardly from and move with top wall 16a. One of the side walls e.g., 16e may be permanently attached to an edge of base 14 and may be hingedly attached to an edge of top wall 16a of lid 16, allowing lid 16 to easily flip between an open and closed position without interference from device components (described below) carried by base 14 and also for convenient mounting of connectors 22a and 22b within sterilization chamber 12. In another embodiment (not shown), the lid 16 may be a completely detachable lid and may be secured in a snap-fit manner to base 14. Other lids known in the art may be used without departing from the scope of the disclosure.

The footprint of lid 16 may correspond with the perimeter of the base 14. For instance, if the perimeter of the base 14 is a rectangular shape, the footprint of lid 16 will be a corresponding rectangular shape to cover the entirety of the base 14.

Lid 16 may include slots 24 in opposed sidewalls 16b and 16c configured to receive tubing when lid 16 is placed in its closed configuration.

As also shown in Fig. 3, sterilization device 10 may include one or more holders 26 carried by base 14 configured to receive connectors 22a, 22b. In one embodiment, the base unit includes two holders 26 initially positioned at opposite ends of base 14 within sterilization chamber 12. Preferably, holders 26 are positioned on either side of a light source 28 in base 14.

The holders 26 may be configured to receive a reusable connector(s) 22a, 22b. In one embodiment, holders 26 may include slots such as U-shaped slots 29 for receiving collars 25, 27 (shown in Fig. 2) of connectors 22a, 22b.

To establish a sterile flow path, holders 26 (with connectors mounted thereon) are moveable towards each other. Movement of holders 26 may be initiated by actuator 20 coupled to holders 26. In one embodiment, the actuator 20 may be a trigger. In another embodiment, the actuator 20 may be a button or a set of buttons. For example, by pressing actuator/trigger 20 to a first position, holders 26 may be moved towards one another to a first position, connecting the reusable connectors 22a, 22b above the light source 28. Again, by moving actuator 20 to a second position, the moveable holders 26 may be moved even closer to one another, opening a fluid pathway between the reusable connectors 22a, 22b.

The actuator 20 may be attached to the device 10 using a pin and hole system. For instance, the actuator 20 may contain a hole in the upper portion. A pin is then inserted through the hole to secure the actuator 20 to the handle 18. This allows the actuator 20 to rotate about the pin in the handle 18. Other methods of attaching the actuator 20 to the device may be used without departing from the scope of the disclosure.

In one embodiment, the actuator 20 is configured to move the holders 26 using a linkage system that will be known to those of skill in the art. For example, actuator 20 may turn a gear which engages two racks configured to move holders 26 closer together. In another embodiment, the actuator 20 may turn a cam which displaces internal stops to allow for linear springs to move the holders 26 towards one another. In yet another embodiment, the actuator 20 could drive two lead screws or roller screws together to move the connectors 26 towards one another.

In another embodiment, the actuator 20 may be a button that sends a signal to a controller configured to move the holders 26. The controller may control motor driven movements within the device 10. In one instance, a single button may be used to move the holders 26. The button may be pressed a first time to send a first signal to the controller to move the holders 26 to a first position above the light source 28. Once the holders 26 are in the first position, the light source 28 may be activated for a preset amount of time. The button may then be pressed a second time to send a second signal to the controller to move the holders 26 into the second position, opening the fluid pathway. In another example, the button may activate the sterilization process by activating a controller associated with a timer. Once the button is pressed, the controller may move the holders 26 into a first position where they are sterilized for a preset amount of time. After sufficient sterilization, the controller may automatically move the holders 26 to a second position, opening the fluid pathway. The controller may then return holders 26 to their initial positions after a preset amount of time or by the user pressing the button a second time sending a second signal to the controller. In another instance, a two-button system may be utilized, where a first button may be pressed to move the holders 26 into the first position, and the second button may be pressed to move the holders 26 into the second position.

Sterilization chamber 12 includes or may be otherwise associated with a light source for providing sterilizing light to sterilization chamber 12. The light source 28 may be housed within base unit 14 and/or upper region of handle 18, such that the connectors 22a, 22b are irradiated from below. In one embodiment, the inner surface of top wall 16a of lid 16 may be coated with a reflective material to provide more uniform irradiation to connectors 22a, 22b and the flow path established by the connectors. For example, inner surface 16a may have a mirror finish. In one embodiment, the light source 28 is located in the center of the base 14. In another embodiment, the light source 28 may span the entire area between the holders 26.

In yet another embodiment, the light source 28 may be placed anywhere inside the sterilization chamber 12, such that the reusable connectors 22a, 22b will be irradiated by the light source 28. For instance, the light source 28 could be associated with the lid 16, such that the light source 28 would irradiate the reusable connectors 22a, 22b from above.

The light source 28 may be, but is not limited to being, an LED light source emitting UV-C light. In an embodiment, the light source 28 may be a UV-C germicidal bulb. Other sterilizing light sources known in the art may be used without departing from the scope of the disclosure. Device 10 may further include a power supply for powering light source 28. Power supply may be housed in handle 18. In one embodiment, the power supply may be a rechargeable battery. In another embodiment, the power supply may be a cord to be plugged into an electrical socket in a wall. The cord may be long enough to allow for mobile use of the device 10.

The hand-held device 10 may be used to create a temporary sterile connection flow path between a source of fluid and other containers. For instance, it may be desirable to transfer fluid from a source container with a large volume of fluid to many smaller containers. In another aspect, the device may be used to take samples from large biological processes requiring sterile pathways.

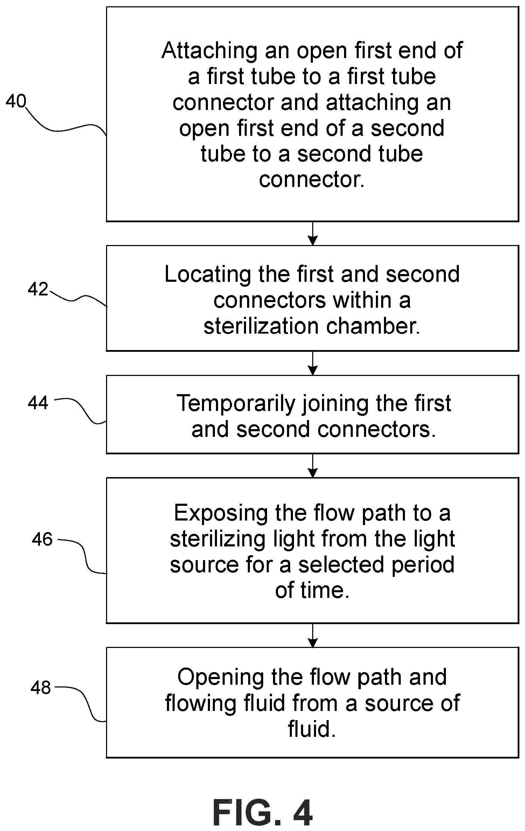

Sterilization of connectors using hand-held device 10 is further shown in Fig. 4, with steps 40-48 setting forth the connection and sterilization steps. At step 40 the user will attach a first end of a first tube 30a to the first reusable connector 22a and a first end of a second tube 30b to the second connector 22b. The connectors 22a, 22b may be attached to the tube using a barb fitting located at the proximal end of the connector. Other methods known in the art may be used to connect the reusable connectors to tubing without departing from the scope of the disclosure. For instance, the reusable connectors 22a and 22b may be connected by, for example, but not limited to, using a luer fitting or gluing the tube to the end of the reusable connector. In another instance, the reusable connectors 22a and 22b may be pre-attached to a short length of tubing to enable the reusable connectors 22a and 22b to be sterile docked to the desired tubing set. This would allow for the reusable connectors 22a and 22b and the pre-attached tubing to be sterilized during manufacturing and to ensure a closed system when using the device 10.

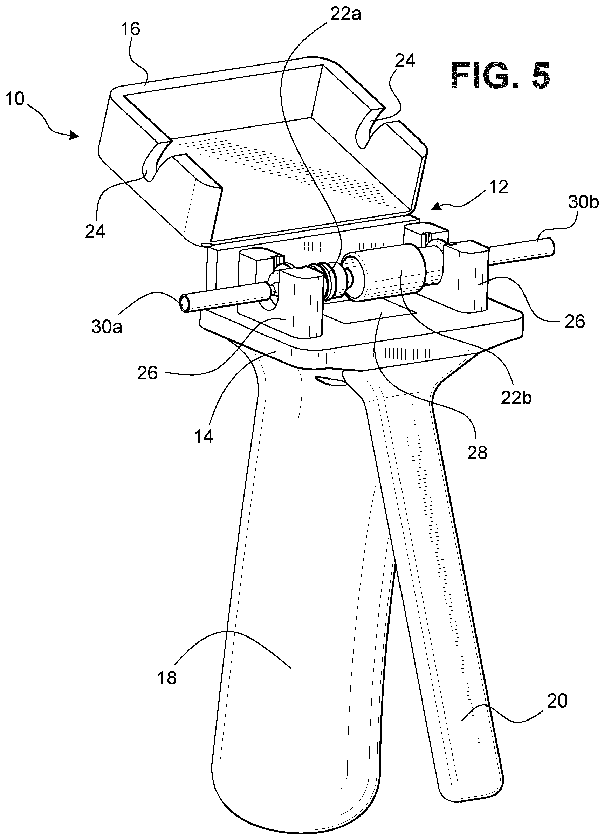

Once the reusable connectors 22a, 22b are attached to the tubing ends 30, at step 42 the connectors 22a, 22b are located within the sterilization chamber 12 by mounting them into a holder 26, as seen in Fig. 5. In one embodiment, the first tubing connector 22a is placed in a first holder 26 within the sterilization chamber 12 and the second connector 22b is placed in a second holder 26 within the sterilization chamber 12. After the connectors 22a, 22b are placed into holders 26, the lid 16 of the separation chamber 12 may be closed.

At step 44 the connectors 22a, 22b are temporarily joined together. To join the connectors 22a, 22b, the actuator 20 is moved/pressed to a first position, moving the holders 26 towards one another such that connector 22b enters the open end of connector 22a. Seal members (O-rings 23) create an air-tight seal between connectors 22a and 22b. Furthermore, while in the first position, the light source 28 is activated. The actuator 20 may directly activate the light source 28. The actuator 20 is kept in the first position for a selected period of time, for example, until the flow path established by the connectors 22a, 22b is sufficiently exposed to light and the flow path is sterilized. In one embodiment where the sterilizing medium is UV-C and the connector housings are made of cyclic olefin copolymer, the duration of irradiation by the light source may be anywhere between 0.1 seconds and 300 seconds. It will be understood that the duration of irradiation may depend, at least in part, on the light intensity.

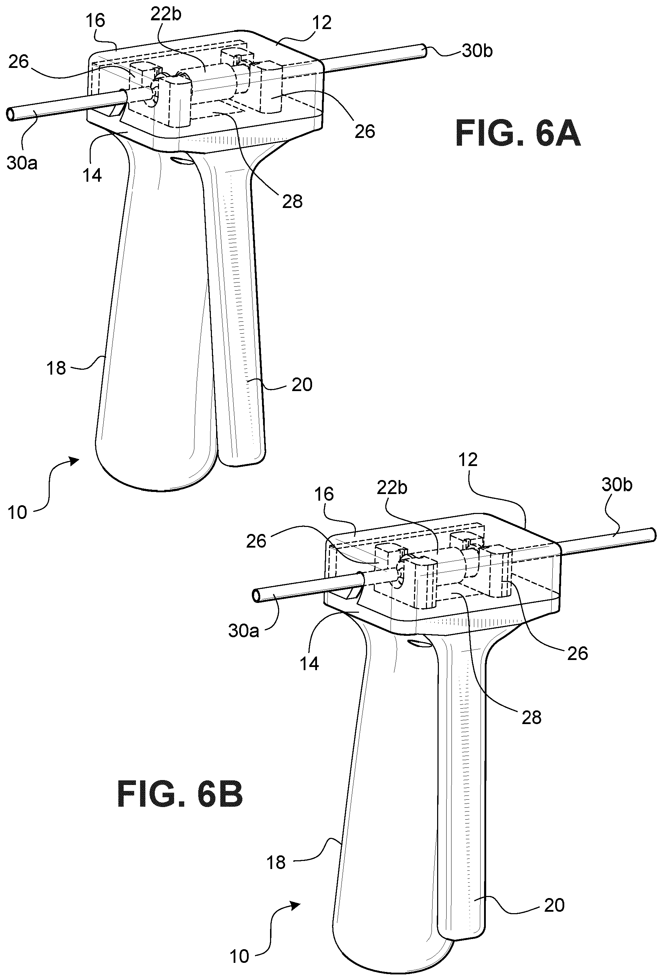

Fig. 6A shows the hand-held device 10 with the lid 16 closed and the actuator 20 depressed to a first position, where the actuator 20 is moved closer to the handle 18. It will be understood that the lid 16 in Fig. 6A is shown as transparent only for the purpose of showing the inside of the sterilization chamber 12 when the lid 16 is closed. Lid 16 is generally made of an opaque material.

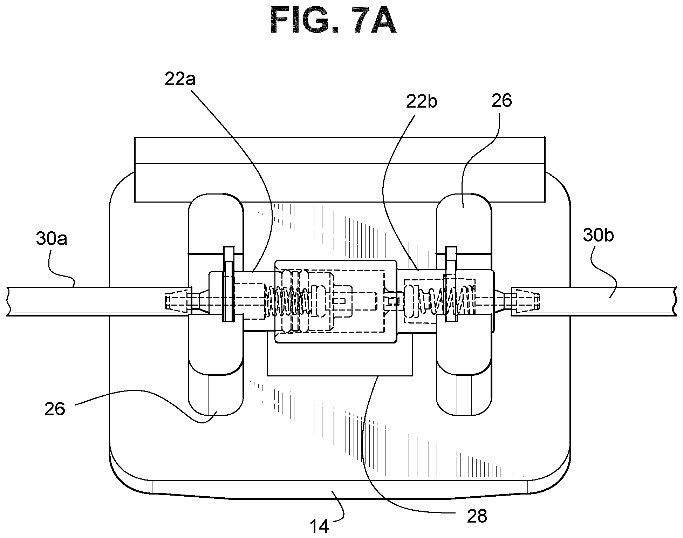

Fig. 7A shows a top perspective view of the base 14 when the actuator 20 is moved to a first position, as in Fig. 6A. In the first position, holders 26 have moved from a starting position near the ends of the base 14, to a position closer towards one another to connect connectors 22a, 22b above the light source 28.

After the flow path is sufficiently irradiated and sterilized, at step 48 the actuator 20 is further depressed towards the handle 18 to a second position. In the second position, the light source is turned off and the connectors 22a, 22b are moved closer together to engage the O-ring face seals of the spring-plunger system, opening the flow path. With an open flow path between processing systems established, fluid from one system, for example a source of fluid, may flow through the sterilized connection and into a second system.

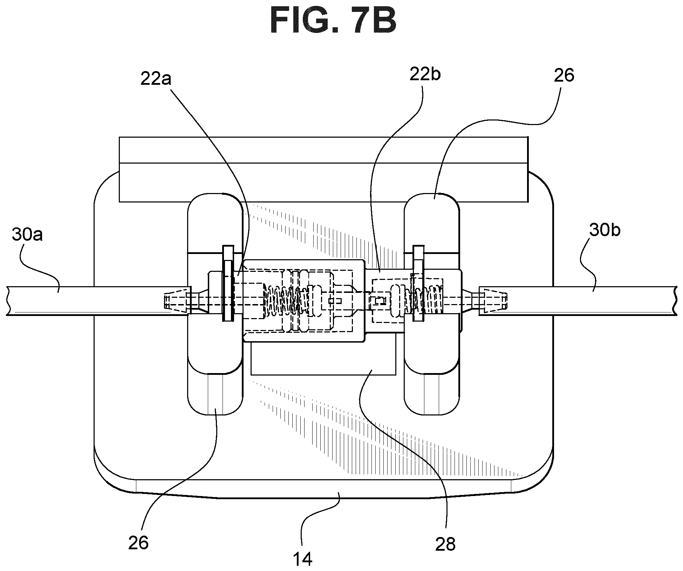

Fig. 6B shows the hand-held device 10 with the actuator 20 moved to a second position. Fig. 7B shows a top perspective view of the base 14 when the holders 26 have been moved to a second, open flow position. In the second position, holders 26 move from the first position to a second position closer towards one another to open the fluid path between connectors 22a, 22b.

Once the desired transfer of fluid through the sterilized flow path is complete, the actuator 20 may be released, returning it to its starting position. By releasing the actuator 20, the holders 26 are returned to their starting positions near the ends of the base 14, thus closing the fluid pathway and disconnecting the connectors 22a, 22b. At this point, the lid 16 may be opened to remove the reusable connectors 22a, 22b from the device 10. Both connectors 22a, 22b can be removed from the device. Alternatively, a single connector 22 may be removed and replaced with a new connector attached to a tube in preparation to create a new sterile pathway.

## Claims

1. A hand-held sterilization device (10) comprising:
a sterilization chamber (12) defined by a base unit (14) and a lid (16);
a first moveable holder (26) and a second moveable holder (26) within said sterilization chamber (12), wherein the first moveable holder and the second moveable holder are configured to hold tubing connectors (22a, 22b);
a handle (18) supporting said sterilization chamber (12);
a light source (28) associated with said sterilization chamber (12); and
an actuator (20) configured to cause movement of said first and second moveable holders (26).

2. The sterilization device of Claim 1, wherein the lid is attached to the base unit and wherein the lid (16) comprises slots (24) configured to receive tubing extending from the tubing connectors (22a, 22b) when the lid is in a closed position.

3. The sterilization device of any one of Claims 1-2 wherein the first and second holders (26) are moveable towards each other.

4. The sterilization device of Claim 3, wherein the first and second holder (26) are moveable towards each other to a first position for sterilization of the tubing connectors and a second position to open a fluid pathway between the tubing connectors (22a, 22b).

5. The sterilization device of any one of Claims 1-4, wherein the first holder and the second holder (26) comprise a groove (29) configured to receive the tubing connectors (22a, 22b).

6. The sterilization device of any one of Claims 4-5, wherein the actuator (20) is a trigger on said handle (18), wherein said trigger is movable to a first position for moving said first and second holders into the holder first position and a second position for moving the first and second holders into a second position.

7. The sterilization device of any one of Claims 4-5, wherein said actuator (20) is a button on the handle (18), said button being configured to move the first and second holders into the first position when said button is pressed a first time and to move the first and second holders into the second position when said button is pressed a second time.

8. The sterilization device of any one of Claims 4-5, wherein said actuator (20) comprises a first and second button on the handle (18), said first button being configured to move the first and second holders to the first position and said second button being configured to move the first and second holders into the second position.

9. A system for providing a sterile fluid pathway connection comprising:
a first reusable tube connector (22a) configured to receive a first tube and a second reusable tube connector (22b) configured to receive a second tube;
and a hand-held sterilization device (10) according to any of the preceding claims.

10. The system of Claim 9, wherein the first reusable tube connector is configured to receive the second reusable tube connector to provide a fluid pathway connection.

11. The system of any one of Claims 9-10, wherein said first moveable holder and second moveable holder are moveable towards each other.

12. The system of Claim 11, wherein the actuator is moveable to a first position to move the first and second holders to a first position and to a second position to open a fluid pathway.

13. A method for transferring fluid in a sterile manner comprising:
attaching an open first end of a tube to a first tube connector (22a) wherein said tube includes a second end in flow communication with a source of fluid;
attaching an open first end of a second tube to a second tube connector (22b);
locating said first and second connectors (22a, 22b) within a sterilization chamber (12) carried by a handle (18) of a hand-held device as defined in any of the claims 1-8, wherein said chamber (12) receives light from a light source (28) associated with said device;
placing the first tube connector (22a) into a first holder (26) and the second tube connector (22b) into a second holder (26) within the sterilization chamber (12);
temporarily joining said first and second connectors (22a and 22b) to establish a flow path between said first and second tubes;
exposing at least said flow path to a sterilizing light from said light source (28) for a selected period of time;
opening the flow path and flowing fluid from said source of fluid through said flow path; and
separating said first and second connectors.

14. The method of Claim 13, further comprising moving an actuator (20) to a first position to establish to establish an air-tight seal between the first connector and second connector and moving said actuator to a second position to open the flow path.

## Patentansprüche

1. Handgehaltene Sterilisationsvorrichtung (10), umfassend:
eine Sterilisationskammer (12), die durch eine Basiseinheit (14) und einen Deckel (16) definiert ist;
einen ersten bewegbaren Halter (26) und einen zweiten bewegbaren Halter (26) innerhalb der Sterilisationskammer (12), wobei der erste bewegbare Halter und der zweite bewegbare Halter dazu konfiguriert sind, Schlauchverbinder (22a, 22b) zu halten;
einen Griff (18), der die Sterilisationskammer (12) trägt;
eine Lichtquelle (28), die mit der Sterilisationskammer (12) assoziiert ist; und
einen Aktor (20), der dazu konfiguriert ist, eine Bewegung des ersten und des zweiten bewegbaren Halters (26) zu bewirken.

2. Sterilisationsvorrichtung nach Anspruch 1, wobei der Deckel an der Basiseinheit angebracht ist und wobei der Deckel (16) Schlitze (24) umfasst, die dazu konfiguriert sind, sich von den Schlauchverbindern (22a, 22b) erstreckende Schläuche aufzunehmen, wenn sich der Deckel in einer geschlossenen Stellung befindet.

3. Sterilisationsvorrichtung nach einem der Ansprüche 1-2, wobei der erste und der zweite Halter (26) aufeinander zu bewegbar sind.

4. Sterilisationsvorrichtung nach Anspruch 3, wobei der erste und der zweite Halter (26) in eine erste Stellung zum Sterilisieren der Schlauchverbinder und in eine zweite Stellung zum Öffnen eines Fluidleitungsleitungspfads zwischen den Schlauchverbindern (22a, 22b) aufeinander zu bewegbar sind.

5. Sterilisationsvorrichtung nach einem der Ansprüche 1-4, wobei der erste Halter und der zweite Halter (26) eine Nut (29) umfassen, die dazu konfiguriert ist, die Schlauchverbinder (22a, 22b) aufzunehmen.

6. Sterilisationsvorrichtung nach einem der Ansprüche 4-5, wobei der Aktor (20) ein Auslöser an dem Griff (18) ist, wobei der Auslöser in eine erste Stellung zum Bewegen des ersten und des zweiten Halters in die erste Halterstellung und eine zweite Stellung zum Bewegen des ersten und des zweiten Halters in eine zweite Stellung bewegbar ist.

7. Sterilisationsvorrichtung nach einem der Ansprüche 4-5, wobei der Aktor (20) eine Taste an dem Griff (18) ist, wobei die Taste dazu konfiguriert ist, den ersten und den zweiten Halter in die erste Stellung zu bewegen, wenn die Taste ein erstes Mal gedrückt wird, und den ersten und den zweiten Halter in die zweite Stellung zu bewegen, wenn die Taste ein zweites Mal gedrückt wird.

8. Sterilisationsvorrichtung nach einem der Ansprüche 4-5, wobei der Aktor (20) eine erste und eine zweite Taste an dem Griff (18) umfasst, wobei die erste Taste dazu konfiguriert ist, den ersten und den zweiten Halter in die erste Stellung zu bewegen, und die zweite Taste dazu konfiguriert ist, den ersten und den zweiten Halter in die zweite Stellung zu bewegen.

9. System zum Bereitstellen einer sterilen Fluidleitungspfadverbindung, umfassend:
einen ersten wiederverwendbaren Schlauchverbinder (22a), der dazu konfiguriert ist, einen ersten Schlauch aufzunehmen, und einen zweiten wiederverwendbaren Schlauchverbinder (22b), der dazu konfiguriert ist, einen zweiten Schlauch aufzunehmen; und
eine handgehaltene Sterilisationsvorrichtung (10) nach einem der vorangehenden Ansprüche.

10. System nach Anspruch 9, wobei der erste wiederverwendbare Schlauchverbinder dazu konfiguriert ist, den zweiten wiederverwendbaren Schlauchverbinder aufzunehmen, um eine Fluidleitungspfadverbindung bereitzustellen.

11. System nach einem der Ansprüche 9-10, wobei der erste bewegbare Halter und der zweite bewegbare Halter aufeinander zu bewegbar sind.

12. System nach Anspruch 11, wobei der Aktor in eine erste Stellung bewegbar ist, um den ersten und den zweiten Halter in eine erste Stellung und in eine zweite Stellung zu bewegen, um einen Fluidleitungspfad zu öffnen.

13. Verfahren zum Übertragen von Fluid auf sterile Weise, umfassend:
Anbringen eines offenen ersten Endes eines Schlauchs an einem ersten Schlauchverbinder (22a), wobei der Schlauch ein zweites Ende in Strömungsverbindung mit einer Quelle von Fluid beinhaltet;
Anbringen eines offenen ersten Endes eines zweiten Schlauchs an einem zweiten Schlauchverbinder (22b);
Positionieren des ersten und des zweiten Verbinders (22a, 22b) in einer von einem Griff (18) einer handgehaltenen Vorrichtung, wie in einem der Ansprüche 1-8 definiert, getragenen Sterilisationskammer (12), wobei die Kammer (12) Licht von einer mit der Vorrichtung assoziierten Lichtquelle (28) empfängt;
Platzieren des ersten Schlauchverbinders (22a) in einen ersten Halter (26) und des zweiten Schlauchverbinders (22b) in einen zweiten Halter (26) in der Sterilisationskammer (12);
vorübergehendes Verbinden des ersten und des zweiten Verbinders (22a und 22b), um einen Strömungspfad zwischen dem ersten und dem zweiten Schlauch zu bilden;
Beaufschlagen mindestens des Strömungspfads mit einem Sterilisationslicht von der Lichtquelle (28) für einen ausgewählten Zeitraum;
Öffnen des Strömungspfads und Leiten von Fluid von der Quelle von Fluid durch den Strömungspfad; und
Trennen des ersten und des zweiten Verbinders.

14. Verfahren nach Anspruch 13, ferner umfassend das Bewegen eines Aktors (20) in eine erste Stellung, um eine luftdichte Dichtung zwischen dem ersten Verbinder und dem zweiten Verbinder zu bilden, und Bewegen des Aktors in eine zweite Stellung, um den Strömungspfad zu öffnen.

## Revendications

1. Dispositif de stérilisation portatif (10) comprenant :
une chambre de stérilisation (12) définie par une unité de base (14) et un couvercle (16) ;
un premier support mobile (26) et un second support mobile (26) à l'intérieur de ladite chambre de stérilisation (12), dans lequel le premier support mobile et le second support mobile sont configurés pour supporter des raccords de tuyauterie (22a, 22b) ;
une poignée (18) supportant ladite chambre de stérilisation (12) ;
une source de lumière (28) associée à ladite chambre de stérilisation (12) ; et
un actionneur (20) configuré pour provoquer un mouvement desdits premier et second supports mobiles (26).

2. Dispositif de stérilisation selon la revendication 1, dans lequel le couvercle est fixé à l'unité de base et dans lequel le couvercle (16) comprend des fentes (24) configurées pour recevoir une tuyauterie s'étendant à partir des raccords de tuyauterie (22a, 22b) lorsque le couvercle est dans une position fermée.

3. Dispositif de stérilisation selon l'une quelconque des revendications 1 à 2 dans lequel les premier et second supports (26) sont mobiles l'un en direction de l'autre.

4. Dispositif de stérilisation selon la revendication 3, dans lequel les premier et second supports (26) sont mobiles l'un en direction de l'autre vers une première position pour la stérilisation des raccords de tuyauterie et une seconde position pour ouvrir un trajet de fluide entre les raccords de tuyauterie (22a, 22b).

5. Dispositif de stérilisation selon l'une quelconque des revendications 1 à 4, dans lequel le premier support et le second support (26) comprennent une rainure (29) configurée pour recevoir les raccords de tuyauterie (22a, 22b).

6. Dispositif de stérilisation selon l'une quelconque des revendications 4 à 5, dans lequel l'actionneur (20) est un déclencheur sur ladite poignée (18), dans lequel ledit déclencheur est mobile vers une première position pour déplacer lesdits premier et second supports dans la première position de support et une seconde position pour déplacer les premier et second supports dans une seconde position.

7. Dispositif de stérilisation selon l'une quelconque des revendications 4 à 5, dans lequel ledit actionneur (20) est un bouton sur la poignée (18), ledit bouton étant configuré pour déplacer les premier et second supports dans la première position lorsque ledit bouton est pressé une première fois et pour déplacer les premier et second supports dans la seconde position lorsque ledit bouton est pressé une seconde fois.

8. Dispositif de stérilisation selon l'une quelconque des revendications 4 à 5, dans lequel ledit actionneur (20) comprend des premier et second boutons sur la poignée (18), ledit premier bouton étant configuré pour déplacer les premier et second supports vers la première position et ledit second bouton étant configuré pour déplacer les premier et second supports dans la seconde position.

9. Système de fourniture d'un raccord de trajet de fluide stérile comprenant :
un premier raccord de tuyau réutilisable (22a) configuré pour recevoir un premier tuyau et un second raccord de tuyau réutilisable (22b) configuré pour recevoir un second tuyau ;
et un dispositif de stérilisation portatif (10) selon l'une quelconque des revendications précédentes.

10. Système selon la revendication 9, dans lequel le premier raccord de tuyau réutilisable est configuré pour recevoir le second raccord de tuyau réutilisable pour fournir un raccord de trajet de fluide.

11. Système selon l'une quelconque des revendications 9 à 10, dans lequel lesdits premier support mobile et second support mobile sont mobiles l'un en direction de l'autre.

12. Système selon la revendication 11, dans lequel l'actionneur est mobile vers une première position pour déplacer les premier et second supports vers une première position et vers une seconde position pour ouvrir un trajet de fluide.

13. Procédé de transfert de fluide d'une manière stérile, le procédé comprenant :
la fixation d'une première extrémité ouverte d'un tuyau à un premier raccord de tuyau (22a) dans lequel ledit tuyau inclut une seconde extrémité en communication fluidique avec une source de fluide ;
la fixation d'une première extrémité ouverte d'un second tuyau avec un second raccord de tuyau (22b) ;
la localisation desdits premier et second raccords (22a, 22b) à l'intérieur d'une chambre de stérilisation (12) supportée par une poignée (18) d'un dispositif portatif tel qu'il est défini dans l'une quelconque des revendications 1 à 8, dans lequel ladite chambre (12) reçoit de la lumière d'une source de lumière (28) associée audit dispositif ;
le placement du premier raccord de tuyau (22a) dans un premier support (26) et du second raccord de tuyau (22b) dans un second support (26) à l'intérieur de la chambre de stérilisation (12) ;
la jonction temporaire desdits premier et second raccords (22a et 22b) pour établir un trajet d'écoulement entre lesdits premier et second tuyaux ;
l'exposition au moins dudit trajet d'écoulement à une lumière de stérilisation provenant de ladite source de lumière (28) pendant une période sélectionnée ;
l'ouverture du trajet d'écoulement et l'écoulement de fluide à partir de ladite source de fluide à travers ledit trajet d'écoulement ; et
la séparation desdits premier et second raccords.

14. Procédé selon la revendication 13, comprenant en outre le déplacement d'un actionneur (20) vers une première position pour établir pour établir un joint étanche à l'air entre le premier raccord et le second raccord et déplacer ledit actionneur vers une seconde position pour ouvrir le trajet d'écoulement.
